# FASCICULE DE BREVET EUROPEEN

(11) **EP 2 782 625 B1**
(45) Date de publication et mention de la délivrance du brevet: **03.04.2019**
(21) Numéro de dépôt: 11801770.6
(22) Date de dépôt: 25.11.2011
(51) Int. Cl.: A61M 5/32

(54) **DISPOSITIF DE RECUPERATION D'UNE AIGUILLE**
ANORDNUNG ZUM ENTFERNEN EINER NADEL
NEEDLE REMOVAL DEVICE

(43) Date de publication de la demande: 01.10.2014
(73) Titulaire: Avenir Performance Europeenne Medical, 77570 Chateau Landon (FR)
(72) Inventeur: SEGUELAS, Etienne, F-89140 Lixy (FR); TEYSSONNEYRE, Marc, F-89150 Vallery (FR)
(74) Mandataire: Rhein, Alain
(86) Numéro de dépôt international: PCT/FR2011/052762
(87) Numéro de publication internationale: WO 2013/076382

(56) Documents cités:
- EP-A1- 1 138 338
- WO-A1-92/13585
- WO-A1-94/05352
- WO-A1-2010/119271
- US-A- 4 494 652
- US-A- 5 209 738
- US-A1- 2002 068 908
- US-A1- 2003 038 046
- US-A1- 2008 221 516

## Description

### Domaine technique et état de l'art

L'invention concerne un dispositif de récupération d'un outil acéré tel qu'une aiguille, outil adapté pour être monté sur un porte-outil tel qu'une seringue pour réaliser une effraction tégumentaire. L'outil acéré présente généralement une extrémité de fixation, sensiblement cylindrique, permettant de solidariser l'outil sur le porte-outil, par exemple par vissage ou plus généralement par un emboîtement de type mâle-femelle.

Les aiguilles sont largement utilisées dans le domaine médical, en milieu professionnel (hôpital, etc.) mais également à titre privé pour le traitement de maladies chroniques telles que le diabète.

Un problème récurent concerne la récupération en toute sécurité de ces aiguilles, ou plus généralement de tout outil acéré utilisé notamment dans le domaine de la santé.

Il s'agit d'une part de désolidariser l'aiguille de la seringue pour pouvoir utiliser la seringue avec une nouvelle aiguille, et d'autre part de permettre la manipulation et le transport en toute sécurité de l'aiguille ou de l'outil usagé vers un lieu de collecte approprié. En toute sécurité signifie ici sans risque de blessure physique et sans risque de contamination bactérienne ou microbiologique pour la personne qui manipule l'aiguille et la seringue. US 2003/0038046 A1, US 2002/0068908 A1, WO 94/05352 et WO 92/13585 divulguent des dispositifs de récupération de l'état de la technique.

### Description de l'invention

L'invention est définie dans la revendication indépendante 1.

Le dispositif selon l'invention est utilisé de la manière suivante. Monté sur le porte-outil, l'outil est inséré à force dans l'extrémité d'immobilisation du tube. La section de l'extrémité du tube se déforme pour venir épouser et coincer la forme de l'extrémité de fixation de l'outil. En immobilisant le tube (et non pas l'outil) avec les mains, un opérateur peut ensuite retirer le porte-outil en le dévissant, l'outil restant immobilisé dans la première extrémité du tube. L'opérateur peut ainsi désolidariser l'outil du porte-outil sans toucher à l'outil, en manipulant simplement le porte-outil et le dispositif de désolidarisation du dispositif de récupération selon l'invention.

Selon des variantes, la section de l'extrémité d'immobilisation du tube peut être cylindrique, ou polygonale, un diamètre d'un cercle inscrit dans la section polygonale étant égal ou légèrement inférieur à un diamètre de l'extrémité de fixation de l'outil.

L'expérience montre qu'une section de forme triangulaire donne les meilleurs résultats. En effet, une forme triangulaire se déforme mieux qu'une forme carrée ou pentagonale pour épouser la forme cylindrique de l'extrémité de fixation de l'aiguille lorsque la dite extrémité est enfoncée à force dans l'extrémité d'immobilisation du tube.

Le dispositif de récupération selon l'invention peut être adapté pour des outils dont les extrémités de fixation ont des diamètres différents. La dimension de la section de l'extrémité d'immobilisation est dans ce cas choisie pour le diamètre le plus petit, la déformabilité de la section permettant l'insertion d'outils dont le diamètre de l'extrémité d'immobilisation est plus grand.

Egalement, une face interne de la première extrémité du tube peut présenter au moins un ergot (112) pour immobiliser l'outil en rotation. L'ergot s'étend à l'intérieur du tube, depuis la face interne du tube. Lorsque l'outil est enfoncé à force, l'ergot vient ainsi en appui sur le pourtour de l'extrémité de fixation de l'outil. L'outil est ainsi maintenu encore plus fortement à l'intérieur du tube.

La forme de l'ergot peut être affinée en fonction de la forme extérieure de l'extrémité de fixation de l'outil. Par exemple, pour un outil présentant des nervures sur le pourtour de son extrémité de fixation, l'ergot d'immobilisation peut présenter une section triangulaire dans un plan perpendiculaire à l'axe longitudinal du tube, un angle (a) entre une première arête (113) de la section de l'ergot d'immobilisation et une tangente à la face interne de la première extrémité du tube étant de l'ordre de 90° ou moins lorsqu'il est mesuré dans le sens anti-horaire. Ainsi, la dite première arrête coopère avec une nervure externe présente sur un pourtour de l'extrémité de fixation de l'outil, pour immobiliser en rotation l'outil par rapport au dispositif de récupération. L'ergot vient ainsi renforcer le pouvoir d'immobilisation de l'extrémité du tube.

De préférence, une deuxième extrémité d'insertion (116) du tube a une section plus grande que la section de la première extrémité, et une paroi interne du tube (115) s'étendant entre les deux extrémités du tube forme un moyen de guidage de l'outil à l'intérieur du tube. L'insertion de l'outil dans le tube jusqu'à l'extrémité d'immobilisation est ainsi facilitée.

Le dispositif de désolidarisation peut encore comprendre un moyen de préhension au voisinage de l'extrémité d'insertion du tube pour faciliter la prise en main du tube. Dans un mode de réalisation, le dit moyen de préhension comprend une collerette (130) s'étendant dans un plan perpendiculaire à l'axe longitudinal, un bord interne (131) de la collerette étant solidaire de l'extrémité d'insertion du tube, un bord externe (132) de la collerette étant prolongé par une bague (133) cylindrique s'étendant selon l'axe longitudinal. Les doigts de l'opérateur, positionnés sur la bague, sont ainsi éloignés de l'extrémité d'insertion de l'outil dans le tube, et protégés d'une éventuelle blessure par le bord de la bague.

Le dispositif de récupération peut encore comprendre un moyen de collecte de forme appropriée pour recevoir une extrémité acéré de l'outil immobilisé dans l'extrémité d'immobilisation du tube. Selon un mode de réalisation, le moyen de collecte est un flacon (140), et une face interne de la bague cylindrique ou une face de dessous de la collerette (130) présente des crochets (134) adaptés pour coopérer par clipsage ou vissage avec des rainures (141) correspondantes d'une ouverture du flacon. Un tel flacon peut permettre la récupération de plusieurs outils, de manière sécuritaire. Selon un autre mode de réalisation, le tube (130) est fermé à son extrémité d'immobilisation. Un tel dispositif est dans ce cas à usage unique, le tube permettant le stockage d'un seul outil.

Le dispositif de récupération peut encore comprendre un moyen anti retour (150) au voisinage de l'extrémité d'insertion du tube, pour empêcher un retrait d'un outil inséré dans le tube de désolidarisation. Ainsi, après désolidarisation de l'outil, l'outil est empêché de sortir du tube par le moyen anti-retour lors du retrait du porte-outil. Tout risque de blessure ou de contamination lors du retrait du porte-outil est ainsi supprimé.

Dans un mode de réalisation, le moyen anti-retour peut comprendre une jupe (151) de forme sensiblement conique dont une extrémité de plus grande section est solidaire de l'extrémité d'insertion du tube, la dite jupe comprenant une pluralité de lamelles (152) élastiques s'étendant dans le tube depuis l'extrémité d'insertion. La jupe peut par exemple comprendre 3 à 8 lamelles. Un minimum de quatre lamelles est préféré pour que les lamelles de la jupe présente une résistance mécanique à la flexion suffisante pour retenir en toute circonstance l'outil à l'intérieur du tube lors du retrait du porte outil.

De préférence, l'extrémité de plus grande section de la jupe solidaire de l'extrémité d'insertion du tube forme un repli (153) élastique. Le repli élastique limite la section accessible de l'extrémité d'insertion du tube lorsqu'aucun porte-outil n'est inséré et permet également d'obtenir un effacement des lamelles facilitant l'insertion du porte-outil équipé de l'outil dans le tube du dispositif de désolidarisation.

Dans un mode de réalisation, le moyen anti-retour comprend une collerette (154) s'étendant dans un plan sensiblement perpendiculaire à l'axe longitudinal, l'extrémité de plus grande section de la jupe étant solidaire d'un bord interne de la collerette, une pluralité de crochets de verrouillage (155) s'étendant depuis une face de la collerette dans la direction longitudinale, les dits crochets étant adaptés à coopérer avec des fentes de verrouillage (136) correspondantes de la collerette (130) du moyen de préhension pour solidariser le moyen anti-retour et le moyen de préhension. la collerette et les crochets de verrouillage permettent d'immobiliser la jupe du moyen anti-retour à l'extrémité d'insertion du tube de sorte que les lamelles de la dite jupe s'étendent à l'intérieur du tube.

Enfin, le dispositif de récupération selon l'invention peut encore comprendre un couvercle (160) monté rotatif par rapport au dispositif de désolidarisation entre une position ouverte dans laquelle l'extrémité d'insertion du moyen de désolidarisation est accessible et une position fermée dans laquelle l'extrémité d'insertion du moyen de désolidarisation est inaccessible. Le couvercle permet d'obturer l'extrémité d'insertion et du moyen de désolidarisation lorsqu'il n'est pas utilisé

### Brève description des figures

L'invention sera mieux comprise, et d'autres caractéristiques et avantages de l'invention apparaîtront à la lumière de la description qui suit d'exemples de dispositifs selon l'invention. Ces exemples sont donnés à titre non limitatif. La description est à lire en relation avec les dessins annexés dans lesquels :
- la figure 1 est une vue en coupe d'un porte-outil de type seringue 2, et d'un outil associé de type aiguille 1
- la figure 2 est une vue de face d'un dispositif selon un premier mode de réalisation de l'invention,
- la figure 3 est une vue en perspective d'un dispositif selon un deuxième mode de réalisation de l'invention,
- la figure 4 est une vue éclatée du dispositif de la figure 2,
- la figure 5 est une vue partielle en coupe A-A du dispositif de la figure 2,
- la figure 6 est une vue partielle de dessous du dispositif de la figure 2, coinçant un outil 2
- la figure 7 est une vue d'un détail d'un élément de la figure 6,
- la figure 8 est une vue en coupe d'un dispositif selon le deuxième mode de réalisation de l'invention,
- la figure 9 est une vue en coupe A-A d'un premier élément d'un dispositif selon la figure 2 ou 3, et
- la figure 10 est une vue de dessus d'un deuxième élément d'un dispositif selon la figure 2 ou 3.

### Description de modes de réalisation de l'invention

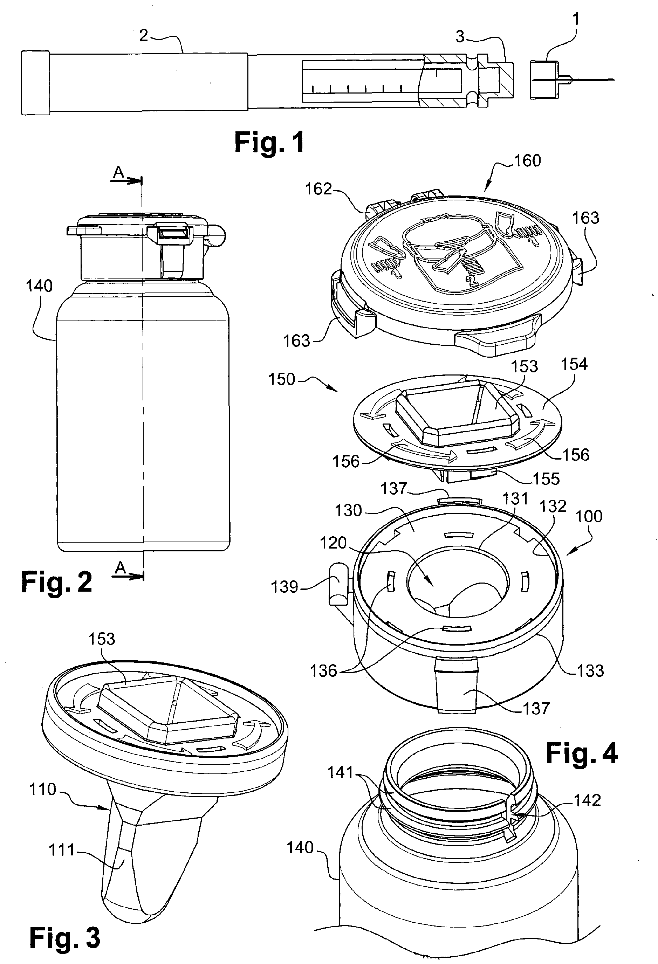

Comme expliqué précédemment, l'invention concerne un dispositif de récupération d'un outil acéré tel qu'une aiguille 1, outil adapté pour être monté sur un porte-outil tel qu'une seringue 2 pour réaliser une effraction tégumentaire, l'outil acéré présentant une extrémité de fixation sensiblement cylindrique 3 (figure 1). Le dispositif selon l'invention peut également être utilisé pour récupérer des outils autres que des seringues, par exemple des bistouris ou plus généralement tout outil présentant une extrémité acérée, piquante, tranchante, etc. susceptible de blesser et de contaminer un opérateur manipulant un tel outil.

Les figures 2, 4, 5, 6 présentent un premier mode de réalisation d'un dispositif selon l'invention, adapté pour la récupération et la collecte de plusieurs outils. Un tel dispositif pourra être utilisé par exemple en milieu hospitalier ou plus généralement par des professionnels du milieu médical utilisant très régulièrement des outils piquants ou tranchants, par exemple plusieurs fois par jours dans un même lieu. Le dispositif des figures 2, 4, 5, 6 comprend un dispositif de désolidarisation 100, un dispositif anti-retour 150, un dispositif de collecte de type flacon 140 et un couvercle 160.

Les figures 3, 8, 10 présentent un deuxième mode de réalisation d'un mode de réalisation d'un dispositif selon l'invention, adapté à un usage unique car permettant la récupération et la collecte d'un unique outil. Un tel dispositif pourra être utilisé par exemple en milieu privé, par exemple par des personnes utilisant des outils piquants ou tranchants occasionnellement et / ou en des lieux différents. Le dispositif des figures 3, 8, 10 comprend un dispositif de désolidarisation 100, fermé, et un dispositif anti-retour 150.

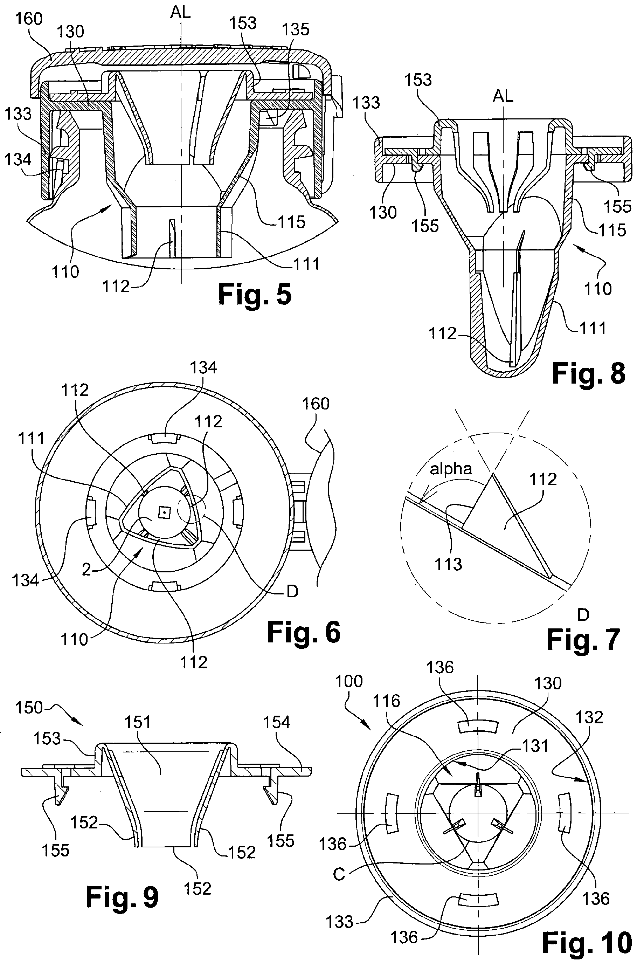

L'élément essentiel d'un dispositif de désolidarisation 100 selon l'invention est un tube 110 de forme sensiblement conique dont une première extrémité d'immobilisation 111 présente, dans un plan perpendiculaire à un axe longitudinal AL du tube, une section de forme appropriée et de déformabilité appropriée pour coincer l'extrémité de fixation 2 de l'outil 1 lorsque le dit outil est inséré à force dans le tube jusqu'à la première extrémité du tube. La première extrémité 111 a de préférence une section polygonale, par exemple carrée ou pentagonale ; l'expérience montre qu'une section triangulaire, comme dans les exemples représentés, est particulièrement efficace pour coincer l'extrémité de fixation 3 de l'outil 1. Un diamètre d'un cercle inscrit C (représenté en trait fin sur la figure 10) dans la section polygonale est égal ou inférieur à un diamètre de l'extrémité de fixation de l'outil pour coincer l'outil.

Une face interne de la première extrémité 111 du tube présente au moins un ergot 112 pour immobiliser l'outil en rotation par rapport à l'axe longitudinal. Dans l'exemple de la figure 6 (vue de dessous, avec outil 2 coincé dans l'extrémité 111), trois ergots sont prévus, un par face de la section triangulaire. Un ergot est détaillé sur la figure 7 (vue de dessous); il présente une section triangulaire dans un plan (plan de la figure 7) perpendiculaire à l'axe longitudinal du tube, un angle alpha entre une première arête 113 de la section de l'ergot 112 d'immobilisation et une tangente à la face interne 114 de la première extrémité du tube étant de l'ordre de 90° ou moins lorsqu'il est mesuré dans le sens anti-horaire. Avec un tel angle alpha l'arrête 113 est adaptée pour coopérer avec une nervure externe (non représentée) présente sur un pourtour de l'extrémité de fixation de l'outil, pour empêcher une rotation de l'outil dans le sens anti-horaire (= sens de dévissage) par rapport au dispositif de récupération. Dans l'exemple de la figure 8, l'ergot 112 a une section triangulaire dans un plan passant par l'axe longitudinal du dispositif de désolidarisation. Ainsi, plus l'outil est enfoncé dans l'extrémité 111 du tube 110, plus il est maintenu fermement par le tube. Une combinaison des deux modes de réalisation des ergots 112 est bien entendu possible.

Une deuxième extrémité 116 d'insertion du tube a une section de surface plus grande qu'une surface de la section de la première extrémité. Ceci facilite l'insertion de la seringue dans le tube 110. La section de la deuxième extrémité 116 peut être polygonale ; elle peut également être circulaire comme dans les exemples représentés. Une paroi interne 115 du tube s'étendant entre les deux extrémités du tube forme un moyen de guidage de l'outil à l'intérieur du tube.

Au voisinage de l'extrémité d'insertion du tube, un moyen de préhension comprend une collerette 130 s'étendant dans un plan perpendiculaire à l'axe longitudinal AL ; un bord interne 131 de la collerette est solidaire de l'extrémité d'insertion 116 du tube et un bord externe 132 de la collerette est prolongé par une bague 133 cylindrique s'étendant selon l'axe longitudinal. La bague facilite la préhension du dispositif, et éloigne les doigts de l'opérateur de la partie acéré de l'outil à isoler.

Le dispositif selon l'invention comprend également un moyen de collecte de forme appropriée pour recevoir une extrémité acérée de l'outil immobilisé dans l'extrémité d'immobilisation du tube. Dans l'exemple des figures 3, 8, 10, le tube 110 du dispositif de désolidarisation est fermé à son extrémité d'immobilisation 111. Dans l'exemple des figures 2, 4, 5, 6, le moyen de collecte est un flacon 140 et une face de dessous de la collerette 130 présente des crochets 134 adaptés pour coopérer par clipsage avec des rainures 141 correspondantes d'une ouverture du flacon. Une face interne de la bague cylindrique comprend également un ergot 135 adapté pour coopérer avec une encoche 142 correspondante de l'ouverture du flacon pour immobiliser en rotation la bague par rapport au flacon. L'ergot 135 empêche la rotation par rapport au flacon du dispositif de désolidarisation clipsé sur le flacon 140. Ainsi, Le dispositif selon l'invention est totalement clos et, une fois l'outil inséré dans le tube 110, la partie acérée de l'outil n'est plus du tout accessible. Tout risque de blessure ou de contamination est ainsi annulé, et l'outil peut être également transporté dans danger.

Dans les exemples représentés encore, le dispositif selon l'invention comprend également un moyen anti retour 150 au voisinage de l'extrémité d'insertion du tube 110, pour empêcher un retrait d'un outil inséré dans le tube de désolidarisation. Le moyen anti-retour comprend une jupe 151 de forme sensiblement conique dont une extrémité de plus grande section est solidaire de l'extrémité d'insertion du tube 110 ; la jupe comprend une pluralité de lamelles 152 élastiques s'étendant dans le tube depuis l'extrémité d'insertion 116. Dans L'exemple des figures 3, 4, 5, 6, 9, la jupe comprend quatre lamelles 152 élastiques. Dans l'exemple de la figure 8, la jupe comprend 6 lamelles (dont 3 seulement sont représentées sur la coupe de la figure 8). La section de la jupe dans un plan perpendiculaire à l'axe longitudinal, notamment au voisinage de l'extrémité d'insertion 116 du tube 110, est fonction essentiellement du nombre de lamelles élastiques. On a ainsi une section carrée sur les figures 3, 4, 5, 6, 9, une section hexagonale dans l'exemple de la figure 8. D'autres variantes ont été réalisées avec 3 à 8 lamelles. Au delà de 8 à 10 lamelles, la jupe devient difficile à réaliser, et les lamelles de petites tailles sont fragiles.

Dans les exemples représentés encore, l'extrémité de plus grande section de la jupe solidaire de l'extrémité d'insertion du tube forme un repli 153 élastique. Le repli 153 apporte une souplesse supplémentaire aux lamelles pour, dans un sens faciliter l'insertion de la seringue et de l'aiguille dans le tube 110, et dans l'autre sens rend plus difficile le retrait intempestif de l'aiguille lorsqu'elle est désolidarisée de la seringue.

En plus de la jupe, le moyen anti-retour comprend une collerette 154 s'étendant dans un plan sensiblement perpendiculaire à l'axe longitudinal AL ; l'extrémité de plus grande section de la jupe est solidaire d'un bord interne de la collerette ; une pluralité de crochets de verrouillage 155 s'étendent depuis une face de dessous de la collerette dans la direction longitudinale et sont adaptés à coopérer avec des fentes de verrouillage 136 correspondantes de la collerette 130 du moyen de désolidarisation pour solidariser le moyen anti-retour et le moyen de préhension.

Le dispositif selon l'invention peut encore comprendre un couvercle 160 monté rotatif par rapport au dispositif de désolidarisation entre une position ouverte dans laquelle l'extrémité d'insertion du moyen de désolidarisation est accessible et une position fermée dans laquelle l'extrémité d'insertion du moyen de désolidarisation est inaccessible. Dans l'exemple des figures 2, 4, 5, 6, une charnière comprenant une partie mâle 139 sur la bague du dispositif de désolidarisation, et une partie femelle 162 sur le couvercle réalise l'articulation entre le couvercle et le reste du dispositif.

Dans le dispositif représenté sur les figures 2, 4, 5, 6, le moyen de préhension 130 comprend également une pluralité de crochets de verrouillage 137 adaptés pour coopérer avec une pluralité de pattes d'accrochage 163 du couvercle pour former un moyen de verrouillage du couvercle en position fermée. Ceci forme une sécurité supplémentaire. Dans les exemples représentés, les crochets 137 sont au nombre de deux, et il est nécessaire d'appuyer simultanément sur les deux crochets pour déverrouiller le couvercle. L'ouverture est ainsi rendue plus complexe, ce qui limite les risques d'ouverture non souhaitée, par des jeunes enfants par exemple.

Dans les exemples représentés sont encore gravées des informations pour l'utilisation du dispositif selon l'invention, notamment :
- des flèches 156 indiquant le sens de rotation de la seringue pour désolidariser l'aiguille de la seringue,
- sur le couvercle, une représentation schématisée de la méthode d'ouverture du couvercle.

## Revendications

1. Dispositif de récupération adapté pour récupérer un outil acéré tel qu'une aiguille (1), outil conçu apte à être monté sur un porte-outil tel qu'une seringue (2) pour réaliser une effraction tégumentaire, l'outil acéré présentant une extrémité de fixation (3) sensiblement cylindrique, le dispositif de récupération comprenant un dispositif de désolidarisation (100) de l'outil du porte-outil, le dispositif de désolidarisation comprenant un tube (110) de forme sensiblement conique dont une première extrémité d'immobilisation (111) présente, dans un plan perpendiculaire à un axe longitudinal du tube, une section polygonale et de préférence triangulaire, **caractérisé en ce que** ladite section est déformable de manière appropriée pour coincer l'extrémité de fixation de l'outil lorsque ledit outil est inséré à force dans le tube (110) jusqu'à la première extrémité d'immobilisation (111) du tube (110), et **en ce qu'**un diamètre d'un cercle inscrit dans la section polygonale est égal ou inférieur à un diamètre de l'extrémité de fixation de l'outil.

2. Dispositif de récupération selon la revendication précédente, dans lequel une face interne de la première extrémité du tube présente au moins un ergot (112) pour immobiliser l'outil en rotation par rapport à l'axe longitudinal.

3. Dispositif selon la revendication 2, dans lequel l'ergot d'immobilisation a une section triangulaire dans un plan perpendiculaire à l'axe longitudinal du tube, un angle (a) entre une première arête (113) de la section de l'ergot (112) d'immobilisation et une tangente à la face interne de la première extrémité du tube étant de l'ordre de 90° ou moins lorsqu'il est mesuré dans le sens anti-horaire, la dite arrête étant adaptée pour coopérer avec une nervure externe présente sur un pourtour de l'extrémité de fixation de l'outil, pour immobiliser en rotation l'outil par rapport au dispositif de récupération.

4. Dispositif selon l'une des revendications précédentes, dans lequel une deuxième extrémité (116) d'insertion du tube a une section de surface plus grande qu'une surface de la section de la première extrémité, et dans lequel une paroi interne du tube (115) s'étendant entre les deux extrémités du tube forme un moyen de guidage de l'outil à l'intérieur du tube.

5. Dispositif selon la revendication 4 dans lequel, le dispositif de désolidarisation comprend un moyen de préhension au voisinage de l'extrémité d'insertion du tube, le dit moyen de préhension comprenant une collerette (130) s'étendant dans un plan perpendiculaire à l'axe longitudinal, un bord interne (131) de la collerette étant solidaire de l'extrémité d'insertion du tube, un bord externe (132) de la collerette étant prolongé par une bague (133) cylindrique s'étendant selon l'axe longitudinal.

6. Dispositif selon l'une des revendications précédentes, comprenant également un moyen de collecte de forme appropriée pour recevoir une extrémité acérée de l'outil immobilisé dans l'extrémité d'immobilisation du tube.

7. Dispositif selon la revendication 6 en combinaison avec la revendication 6, dans lequel le moyen de collecte est un flacon (140), et dans lequel une face interne de la bague cylindrique ou une face de dessous de la collerette présente des crochets (134) adaptés pour coopérer par clipsage ou vissage avec des rainures (141) correspondantes d'une ouverture du flacon.

8. Dispositif selon la revendication 7, dans lequel la face interne de la bague cylindrique comprend également un ergot (135) adapté pour coopérer avec une encoche (142) correspondante de l'ouverture du flacon pour immobiliser en rotation la bague par rapport au flacon.

9. Dispositif selon l'une des revendications précédentes, dans lequel le tube est fermé à son extrémité d'immobilisation.

10. Dispositif selon l'une des revendications 4 à 9 comprenant également un moyen anti retour (150) au voisinage de l'extrémité d'insertion du tube, pour empêcher un retrait d'un outil inséré dans le tube de désolidarisation,

11. Dispositif selon la revendication 10, dans lequel le moyen anti-retour comprend une jupe (151) de forme sensiblement conique dont une extrémité de plus grande section est solidaire de l'extrémité d'insertion du tube, la dite jupe comprenant une pluralité de lamelles (152) élastiques s'étendant dans le tube depuis l'extrémité d'insertion.

12. Dispositif selon la revendication 11 dans lequel l'extrémité de plus grande section de la jupe solidaire de l'extrémité d'insertion du tube forme un repli (153) élastique.

13. Dispositif selon l'une des revendications 10 à 12 en combinaison avec la revendication 6 dans lequel le moyen anti-retour comprend une collerette (154) s'étendant dans un plan sensiblement perpendiculaire à l'axe longitudinal, l'extrémité de plus grande section de la jupe étant solidaire d'un bord interne de la collerette, une pluralité de crochets de verrouillage (155) s'étendant depuis une face de la collerette dans la direction longitudinale, les dits crochets étant adaptés à coopérer avec des fentes de verrouillage (136) correspondantes de la collerette (130) du moyen de préhension pour solidariser le moyen anti-retour et le moyen de préhension.

14. Dispositif selon l'une des revendications précédentes, comprenant également un couvercle (160) monté rotatif par rapport au dispositif de désolidarisation entre une position ouverte dans laquelle l'extrémité d'insertion du moyen de désolidarisation est accessible et une position fermée dans laquelle l'extrémité d'insertion du moyen de désolidarisation est inaccessible.

15. Dispositif selon la revendication 14, dans lequel le moyen de préhension 130 comprend également une pluralité de crochets de verrouillage 137 adaptés pour coopérer avec une pluralité de pattes d'accrochage 163 du couvercle pour former un moyen de verrouillage du couvercle en position fermée.

## Patentansprüche

1. Anordnung zum Entfernen, die ausgelegt ist, um ein spitzes Werkzeug wie z. B. eine Nadel (1) zu entfernen, wobei das Werkzeug als geeignet erachtet wird, um auf einen Werkzeugträger (2) wie z. B. einer Spritze montiert zu sein, um ein Einstechen der Haut durchzuführen, wobei das spitze Werkzeug ein Befestigungsende (3) darstellt, das im Wesentlichen zylindrisch ist, wobei die Anordnung zum Entfernen eine Anordnung zum Trennen (100) des Werkzeugs vom Werkzeugträger umfasst, wobei die Anordnung zum Trennen ein Rohr (110) mit im Wesentlichen konischer Form umfasst, von dem ein erstes Ende zur Immobilisierung (111) auf einer Ebene senkrecht zu einer Längsachse des Rohrs einen polygonalen und vorzugsweise dreieckigen Abschnitt aufweist, **dadurch gekennzeichnet, dass** der Abschnitt auf geeignete Weise verformbar ist, um das Fixierungsende des Werkzeugs einzuklemmen, wenn das Werkzeug mit Kraft in das Rohr (110) bis zu einem ersten Immobilisierungsende (111) des Rohrs (110) eingeführt wird, und dadurch, dass ein Durchmesser eines Kreises, der im polygonalen Abschnitt eingeschrieben ist, gleich wie oder geringer als ein Durchmesser des Befestigungsendes des Werkzeugs ist.

2. Anordnung zum Entfernen nach dem vorhergehenden Anspruch, wobei eine innere Seite des ersten Endes des Rohrs mindestens einen Dorn (112) aufweist, um das Werkzeug in Drehung mit Bezug auf die Längsachse zu immobilisieren.

3. Anordnung nach Anspruch 2, wobei der Dorn zur Immobilisierung einen dreieckigen Abschnitt auf einer senkrechten Ebene zur Längsachse des Rohrs aufweist, wobei Winkel (a) zwischen einer ersten Kante (113) des Abschnitts des Dorns (112) zur Immobilisierung und einer Tangente zur inneren Seite des ersten Endes des Rohrs im Bereich von 90º oder weniger liegt, wenn er entgegen dem Uhrzeigersinn gemessen wird, wobei die Kante ausgelegt ist, um mit einer externen Rippe zusammenzuarbeiten, die auf einem Umfang des Fixierungsendes des Werkzeugs vorhanden ist, um das Werkzeug mit Bezug auf die Anordnung zum Entfernen in Drehung zu immobilisieren.

4. Anordnung nach einem der vorhergehenden Ansprüche, wobei ein zweites Einführungsende (116) des Rohrs einen größeren Flächenschnitt als einen Flächenschnitt des ersten Endes aufweist, und wobei sich eine innere Wand des Rohrs (115) die sich zwischen den zwei Enden des Rohrs erstreckt, ein Mittel zur Führung des Werkzeugs in das innere des Rohrs bildet.

5. Anordnung nach Anspruch 4, wobei die Anordnung zum Trennen ein Mittel zum Vorspannen in der Umgebung des Einführungsendes des Rohrs umfasst, wobei das Vorspannungsmittel einen Kragen (130) umfasst, der sich auf einer senkrechten Ebene zur Längsachse erstreckt, wobei ein innerer Rand (131) des Kragens fest mit dem Einführungsende des Rohrs verbunden ist, wobei ein äußerer Rand (132) des Kragens durch einen zylindrischen Ring (133) verlängert ist, der sich gemäß der Längsachse erstreckt.

6. Anordnung nach einem der vorhergehenden Ansprüche, umfassend auch ein Sammelmittel geeigneter Form, um ein spitzes Ende des immobilisierten Werkzeugs im Immobilisierungsende des Rohrs aufzunehmen.

7. Anordnung nach Anspruch 6 in Kombination mit Anspruch 6, wobei das Sammelmittel ein Kolben (140) ist, und wobei eine innere Seite des zylindrischen Rings oder eine Seite unter dem Kragen Haken (134) aufweist, die ausgelegt sind, um durch Schnappen oder Schrauben mit Nuten (141), die einer Öffnung des Kolbens entsprechen, zusammenzuarbeiten.

8. Anordnung nach Anspruch 7, wobei die innere Seite des zylindrischen Rings auch einen Dorn (135) umfasst, der ausgelegt ist, um mit einer Kerbe (142), die der Öffnung des Kolbens entspricht, zusammenzuarbeiten, um in Drehung den Rings mit Bezug auf den Kolben zu immobilisieren.

9. Anordnung nach einem der vorhergehenden Ansprüche, wobei das Rohr an seinem Immobilisierungsende geschlossen ist.

10. Anordnung nach einem der Ansprüche 4 bis 9, umfassend auch ein Rückschlagmittel (150) in der Umgebung des Einführungsendes des Rohrs, um einen Rückzug eines Werkzeugs, das in das Rohr zum Trennen eingeführt ist, zu verhindern.

11. Anordnung nach Anspruch 10, wobei das Rückschlagmittel einen Mantel (151) in einer im Wesentlichen konischen Form umfasst, von dem ein Ende mit größerem Schnitt fest mit dem Einführungsende des Rohrs verbunden ist, wobei der Mantel eine Vielzahl von elastischen Lamellen (152) umfasst, die sich im Rohr vom Einführungsende erstrecken.

12. Anordnung nach Anspruch 11, wobei das Ende mit dem größeren Schnitt des Mantels, das mit dem Einführungsende des Rohrs fest verbunden ist, eine elastische Falte (153) bildet.

13. Anordnung nach einem der Ansprüche 10 bis 12 in Kombination mit Anspruch 6, wobei das Rückschlagmittel einen Kragen (154) umfasst, der sich auf einer im Wesentlichen senkrechten Ebene zur Längsachse erstreckt, wobei das Ende mit dem größten Schnitt des Mantels fest mit einem inneren Rand des Kragens verbunden ist, wobei sich eine Vielzahl von Verriegelungshaken (155) von einer Seite des Kragens in der Längsrichtung erstreckt, wobei die Haken ausgelegt sind, um mit entsprechenden Verriegelungsschlitzen (136) des Kragens (130) des Vorspannugsmittels zusammenzuarbeiten, um das Rückschlagmittel und das Vorspannungsmittel fest miteinander zu verbinden.

14. Anordnung nach einem der vorhergehenden Ansprüche, umfassend auch einen Deckel (160), der drehend mit Bezug auf die Anordnung zum Trennen zwischen einer offenen Position, in der das Einführungsende des Mittels zum Trennen zugänglich ist, und einer geschlossenen Position, in der das Einführungsende des Mittels zum Trennen nicht zugänglich ist, montiert ist.

15. Anordnung nach Anspruch 14, wobei das Vorspannungsmittel 130 eine Vielzahl von Verriegelungshaken (137) umfasst, die ausgelegt sind, um mit einer Vielzahl von Befestigungfüßen 163 des Deckels zusammenzuarbeiten, um ein Verriegelungsmittel des Deckels in geschlossener Position zu bilden.

## Claims

1. A recovery device suitable for recovering a sharp tool such as a needle (1), the tool being designed able to be mounted on a toolholder such as a syringe (2) for integument puncture, the sharp tool having a substantially cylindrical attachment end (3), the recovery device comprising a device for detaching (100) the tool from the toolholder, the detaching device comprising a substantially conical tube (110), a first immobilizing end (111) of which has, in a plane perpendicular to a longitudinal axis of the tube, a polygonal and preferably triangular section, **characterized in that** said section is deformable appropriately to jam the attachment end of the tool when said tool is inserted forcibly into the tube (110) up to the first immobilizing end (111) of the tube (110), and **in that** a diameter of a circle inscribed in the polygonal section is equal to or smaller than a diameter of the attachment end of the tool.

2. The recovery device according to the preceding claim, wherein an inner face of the first end of the tube has at least one lug (112) for immobilizing the tool in rotation relative to the longitudinal axis.

3. The device according to claim 2, wherein the immobilizing lug has a triangular section in a plane perpendicular to the longitudinal axis of the tube, an angle (a) between a first rim (113) of the section of the immobilizing lug (112) and a tangent to the inner face of the first end of the tube being about 90° or less when it is measured in the counterclockwise direction, said rim being suitable for cooperating with an outer rib present on a perimeter of the fastening end of the tool, to immobilize the tool in rotation relative to the recovery device.

4. The device according to one of the preceding claims, wherein a second end (116) for insertion of the tube has a section with a surface larger than a surface of the section of the first end, and wherein an inner wall of the tube (115) extending between the two ends of the tube forms a guide means of the tool inside the tube.

5. The device according to claim 4, wherein the detaching device comprises a gripping means near the insertion end of the tube, said gripping means comprising a collar (130) extending in a plane perpendicular to the longitudinal axis, an inner edge (131) of the collar being secured to the insertion end of the tube, an outer edge (132) of the collar being extended by a cylindrical ring (133) extending along the longitudinal axis.

6. The device according to one of the preceding claims, also comprising a collection means with a shape appropriate to receive a sharp end of the tool immobilized in the immobilizing end of the tube.

7. The device according to claim 6 combined with claim 6 [sic], wherein the collection means is a bottle (140), and wherein an inner face of the cylindrical ring or a bottom face of the collar has hooks (134) suitable for cooperating by clipping or screwing with corresponding grooves (141) of an opening of the bottle.

8. The device according to claim 7, wherein the inner face of the cylindrical ring also comprises a lug (135) suitable for cooperating with a corresponding notch (142) of the opening of the bottle to immobilize the rotation of the ring relative to the bottle.

9. The device according to one of the preceding claims, wherein the tube is closed at its immobilizing end.

10. The device according to one of claims 4 to 9, also comprising a nonreturn means (150) near the insertion end of the tube, to prevent removal of the tool inserted into the detaching tube.

11. The device according to claim 10, wherein the nonreturn means comprises a skirt (151) with a substantially conical shape, an end of which with a larger section is secured to the insertion end of the tube, said skirt comprising a plurality of resilient strips (152) extending in the tube from the insertion end.

12. The device according to claim 11, wherein the end with a larger section of the skirt secured to the insertion end of the tube forms a resilient fold (153).

13. The device according to one of claims 10 to 12 in combination with claim 6, wherein the nonreturn means comprises a collar (154) extending in a plane substantially perpendicular to the longitudinal axis, the end of larger section of the skirt being secured to an inner edge of the collar, a plurality of locking hooks (155) extending from a face of the collar in the longitudinal direction, said hooks being suitable for cooperating with corresponding locking slits (136) of the collar (130) of the gripping means to secure the nonreturn means and the gripping means.

14. The device according to one of the preceding claims, also comprising a lid (160) mounted rotating relative to the detaching device between an open position in which the insertion end of the detaching means is accessible and a closed position in which the insertion end of the detaching means is inaccessible.

15. The device according to claim 14, wherein the gripping means (130) also comprises a plurality of locking hooks (137) suitable for cooperating with the plurality of hooking tabs (163) of the lid to form a means for locking the lid in the closed position.
